# EUROPÄISCHE PATENTSCHRIFT

(11) **EP 2 596 767 B1**
(45) Veröffentlichungstag und Bekanntmachung des Hinweises auf die Patenterteilung: **18.05.2016**
(21) Anmeldenummer: 12190799.2
(22) Anmeldetag: 31.10.2012
(51) Int. Cl.: A61F 2/95, A61M 25/00

(54) **Freigabevorrichtung zum Lösen eines medizinischen Implantats von einer Einführvorrichtung sowie Einführvorrichtung mit einer Freigabevorrichtung**
Release device for detaching a medical implant from an insertion device and an insertion device comprising a release device
Dispositif de libération pour libérer un implant médical d'un dispositif d'introduction ainsi que dispositif d'introduction avec dispositif de libération

(30) Priorität: 24.11.2011 US 201161563556 P
(43) Veröffentlichungstag der Anmeldung: 29.05.2013
(73) Patentinhaber: Biotronik AG, 8180 Bülach (CH)
(72) Erfinder: Fargahi, Amir, 8180 Bülach (CH)
(74) Vertreter: Galander, Marcus

(56) Entgegenhaltungen:
- WO-A1-2008/124844
- WO-A2-2010/042950
- US-A- 5 776 142
- US-A1- 2005 080 476

## Beschreibung

Die Erfindung betrifft eine Freigabevorrichtung zum Lösen eines medizinischen Implantats von einem Katheter sowie einen Katheter mit einer Freigabevorrichtung zum Freigeben eines medizinischen Implantats zur Implantation in einen tierischen und/oder menschlichen Körper nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents.

Implantate sind zum Einführen in den Körper mit Kathetern verbunden und müssen am Einsatzort genau platziert und definiert freigegeben werden können. Hierzu ist beispielsweise bekannt, das Implantat durch eine Schiebbewegung freizugeben.

US 2005/0080476 offenbart ein Einführsystem für ein medizinisches Gerät sowie zugehörige Komponenten und Verfahren.

Der Erfindung liegt die Aufgabe zugrunde, eine Freigabevorrichtung anzugeben, mit der das gezielte Freigeben eines Implantats verbessert ist.

Eine weitere Aufgabe ist in der Bereitstellung einer entsprechenden Einführvorrichtung zu sehen.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Freigabevorrichtung zum Lösen eines medizinisches Implantats von einer Einführvorrichtung vorgeschlagen, bei welcher das Implantat durch eine Relativbewegung zwischen einem ersten und einem zweiten Einführelement freisetzbar ist, umfassend einen Körper mit einem proximalen Ende, das im Benutzungszustand einem Anwender zugewandt ist und einem distalen Ende, das im Benutzungszustand vom Anwender entfernt ist, wobei zwischen dem proximalen und dem distalen Ende eine Spindel mit wenigstens zwei Geschwindigkeitsbereichen vorgesehen ist, wobei die Spindel zur Erzeugung einer gezielten Relativbewegung zwischen dem ersten und dem zweiten Einführelement der Einführvorrichtung vorgesehen ist.

Vorteilhaft kann ein einfacher Wechsel von schnelles auf langsames Freigeben erfolgen und umgekehrt. Hierzu sind Mechanismen für schnelles auf langsames Freigeben vorgesehen. Die Erfindung ermöglicht eine einfache Handhabung, insbesondere ein Umschalten zwischen den Geschwindigkeitsbereichen. Dies erlaubt eine unkomplizierte, einfache und schnelle Geschwindigkeitsregulierung bei der Freigabe des Implantats. Die Freigabe des Implantats wird präziser und schneller.

Gemäß einer vorteilhaften Ausgestaltung können die Geschwindigkeitsbereiche jeweils entlang einer Längserstreckung der Spindel angeordnet sein. Dies erlaubt eine kompakte und einfache Konstruktion der Freigabevorrichtung.

Gemäß einer vorteilhaften Ausgestaltung können die Geschwindigkeitsbereiche entlang der Längserstreckung hintereinander angeordnet sein. Die Geschwindigkeitsbereiche können übersichtlich angeordnet werden und leicht aktiviert werden.

Dabei ist dem jeweiligen Geschwindigkeitsbereich je ein Wirkelement zugeordnet, das mit dem jeweiligen Geschwindigkeitsbereich zusammenwirkt, um die Relativbewegung zwischen dem ersten und dem zweiten Einführelement der Einführvorrichtung zu bewirken. Insbesondere kann der jeweilige Geschwindigkeitsbereich ein Gewinde aufweisen, wobei die Geschwindigkeitsbereiche voneinander unterschiedliche Gewindesteigungen aufweisen. Vorteilhaft kann das jeweilige Wirkelement ein Betätigungselement aufweisen, das aus dem Gehäuse ragt. Mit besonderem Vorteil kann das Wirkelement eine Mutter mit dem entsprechenden Geschwindigkeitsbereich der Spindel korrespondierender Steigung umfassen. Bei einem Mutternantrieb steht die Drehachse des Zahnrades parallel zur Verschieberichtung der Spindel. Dies erlaubt auf einfache Weise eine Umsetzung von zwei unterschiedlichen Geschwindigkeitsbereichen der Freigabevorrichtung. Eine hohe Präzision bei der Positionierung des Implantats kann durch den Einsatz der Muttern erreicht werden, statt wie im Stand der Technik ein Schieben und Zurückziehen der Einführelemente einzusetzen.

Vorteilhaft kann das Wirkelement gegenüber einem Gehäuse fixiert sein, das wenigstens um die Spindel angeordnet ist. Das Gehäuse kann ein Handgriff der Einführvorrichtung zum Einführen eines medizinischen Implantats sein. Vorteilhaft kann das jeweilige Wirkelement aus dem Gehäuse ragen, womit dieses leicht von außen betätigbar ist.

Gemäß einer günstigen Ausgestaltung kann das Wirkelement wenigstens bereichsweise ein Gewinde umfassen, welches insbesondere im Wirkelement radial verschiebbar ist. Die radiale Verschiebung kann insbesondere durch eine Drehbewegung des Wirkelements erreicht werden.

Gemäß einem weiteren Aspekt der Erfindung wird eine Einführvorrichtung zum Einführen eines medizinischen Implantats vorgeschlagen, welches durch eine Relativbewegung zwischen einem ersten und einem zweiten Einführelement freisetzbar ist, umfassend eine erfindungsgemäße Freigabevorrichtung zum Lösen des medizinisches Implantats, umfassend einen Körper mit einem proximalen Ende, das im Benutzungszustand einem Anwender zugewandt ist und einem distalen Ende, das im Benutzungszustand vom Anwender entfernt ist, wobei zwischen dem proximalen und dem distalen Ende eine Spindel mit wenigstens zwei Geschwindigkeitsbereichen vorgesehen ist, wobei die Spindel zur Erzeugung einer gezielten Relativbewegung zwischen dem ersten und dem zweiten (Einführelement der Einführvorrichtung vorgesehen ist.

Die Einführvorrichtung kann günstigerweise ein Katheter sein. Besonders vorteilhaft kann die Einführvorrichtung zur Montage und Freigabe einer Prothese, einer Herzklappe oder eines Stents eingesetzt werden.

Gemäß einer günstigen Ausgestaltung kann am distalen Ende der Spindel eine Schleusenvorrichtung angeordnet sein, die mit einem äußeren der Einführelemente verbunden ist. Insbesondere kann die Spindel an der Schleusenvorrichtung fixiert sein. Dies erlaubt eine stabile und belastbare Konstruktion.

Gemäß einer günstigen Ausgestaltung kann ein inneres Einführelement durch die Spindel geführt sein. Dies erlaubt eine kompakte Bauweise der Einfiihrvorrichtung.

Die Erfindung ist nachfolgend beispielhaft, anhand von in Zeichnungen dargestellten Ausführungsbeispielen, näher erläutert. Es zeigen in schematischer Darstellung:
- Fig. 1: in einer Seitenansicht eine Ausgestaltung einer Einführvorrichtung und einer Freigabevorrichtung nach der Erfindung mit einem aufgeschnittenen Gehäuse;
- Fig. 2a, 2b: in einer Seitenansicht und Draufsicht eine günstige Ausgestaltung eines Wirkelements mit einem Gewindestück in deaktivertem Zustand (Fig. 2a) und im aktivierten, radial nach innen verschobenem Gewindestück (Fig. 2b); und
- Fig. 3a-3c: in einer Seitenansicht auf die Freigabevorrichtung aus Fig. 1 verschiedene Positionen der Wirkelement zur langsamen Freisetzung eines Implantats;
- Fig. 4: in einer Seitenansicht auf die Freigabevorrichtung aus Fig. 1 eine Position der Wirkelemente zur schnellen Freisetzung eines Implantats.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

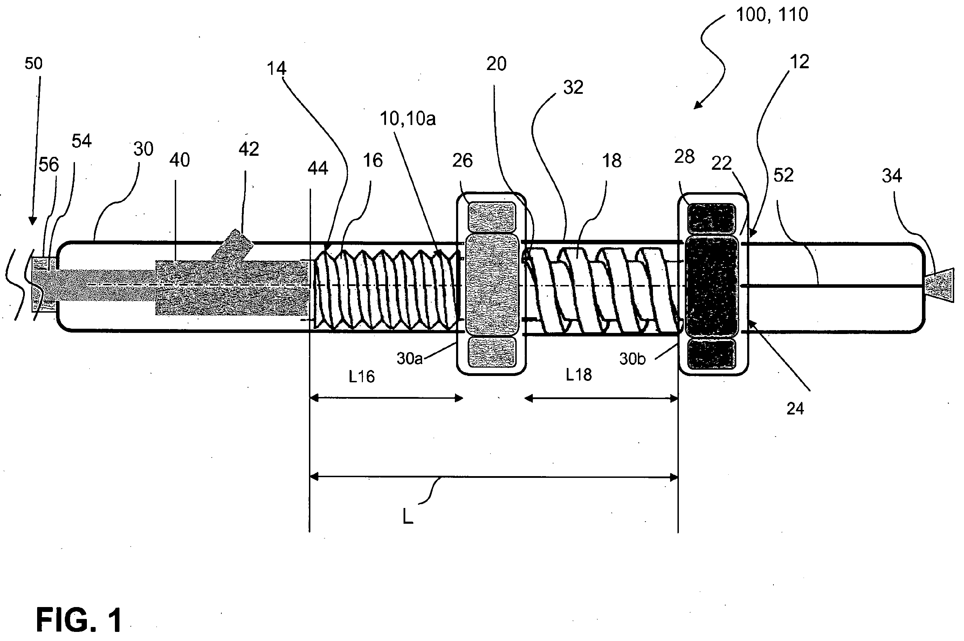

Figur 1 zeigt schematisch in einer Seitenansicht eine günstige Ausgestaltung eines Details einer Einführvorrichtung 110 und einer Freigabevorrichtung 100 nach der Erfindung mit einem aufgeschnittenen Gehäuse 30, welches eine Handgriff der Einführvorrichtung 110 bildet.

Die Einführvorrichtung 110 ist beispielsweise ein Katheter mit einem Schaftbereich 50 mit zwei koaxial angeordneten Einführelementen 52, 54, z.B. einem Innenschaft (Einfuhrelement 52) und einem diesen umgebenden Außenschaft (Einführelement 54), welcher wiederum von einer Außenhülle 56 umgeben sein kann. Die Einführvorrichtung 110 ist nur an ihrem proximalen Ende dargestellt, das einem Anwender zugewandt ist. Das Implantat (nicht dargestellt) ist üblicherweise am distalen Ende des Schaftbereichs 50 zwischen Innenschaft und Außenschaft platziert und soll am Implantationsort im tierischen oder menschlichen Körper freigegeben werden.

Die Freigabevorrichtung 100 dient zum Lösen des medizinischen Implantats von der Einführvorrichtung 110. Das Implantat ist an einem dem Gehäuse 30 entgegengesetzten Ende des Schaftbereichs 50 angeordnet. Das Implantat ist beispielsweise um das innere Einführelement 52 gelegt und wird durch eine Relativbewegung zwischen dem ersten und dem zweiten (Einführelement 52, 54 freigesetzt.

Die Freigabevorrichtung 100 umfasst einen Körper 10 mit einem proximalen Ende 12, das im Benutzungszustand einem Anwender zugewandt ist und einem distalen Ende 14, das im Benutzungszustand vom Anwender entfernt ist. Zwischen den beiden Enden 12, 14 erstreckt sich eine Spindel 10a, die zwei Geschwindigkeitsbereiche 16, 18 aufweist. Der erste Geschwindigkeitsbereich 16 dient zum langsamen Freisetzen und weist ein Gewinde mit geringer Gewindesteigung auf, der zweite Geschwindigkeitsbereich 18 dient zum schnellen Freisetzen und weist ein Gewinde mit einer anderen, nämlich steileren Gewindesteigung auf. Die Spindel 10a dient zur Erzeugung einer gezielten Relativbewegung zwischen dem ersten und dem zweiten Einführelement 52, 54, die schnell oder langsam erfolgen kann, je nachdem, welcher Geschwindigkeitsbereich 16 oder 18 aktiviert ist.

Das Gehäuse 30 kann den Handgriff der Einführvorrichtung 110 bilden und umgibt die Spindel 10a sowie einen Schleusenkörper 40 (auch bekannt als T-Körper). Der Schleusenkörper 40 weist ein Anschlussstück 42, z.B. in Form eines Luer-Locks 42, auf.

Für die auf der Spindel 10a befindlichen Geschwindigkeitsbereiche 16, 18 ist jeweils ein passendes Wirkelement 26, 28, z.B. in Form einer Schraubenmutter, vorgesehen. Die Wirkelemente 26, 28 sind durch hierfür angepasste Gehäusebereiche 30a bzw. 30b von außen zugänglich und sind in diesen Gehäusebereichen 30a, 30b gegenüber dem Gehäuse 30 in Längsrichtung des Gehäuses 30 fixiert.

Das innere Einführelement 52 (Katheter-Innenschaft) ist auf dem Gehäuse mit einem Anschlussstück 34, z.B. in Form eines Luer-Locks, fixiert, wobei das Einführelement 52 durch eine Durchführung 24 in der Spindel 10a geführt ist. Der Schleusenkörper 40 ist mit dem vorderen Gewinde (Geschwindigkeitsbereich 16) an einer Fixierung 44 starr verbunden. Beim Drehen des jeweiligen Wirkelements 26, 28 kann der Außenschaft (Einführelement 54) gegenüber dem Innenschaft (Einführelement 52) bewegt bzw. verschoben werden, indem das jeweilige Wirkelement 26, 28 durch Drehen um die Spindel 10a entlang der axialen Erstreckung L16, L18 des jeweiligen Geschwindigkeitsbereichs 16, 18 bewegt wird. Die Länge jedes Geschwindigkeitsbereichs 16, 18 ist zweckmäßigerweise so bemessen, dass diese mindestens so lang ist wie die freizugebende Länge des Implantats.

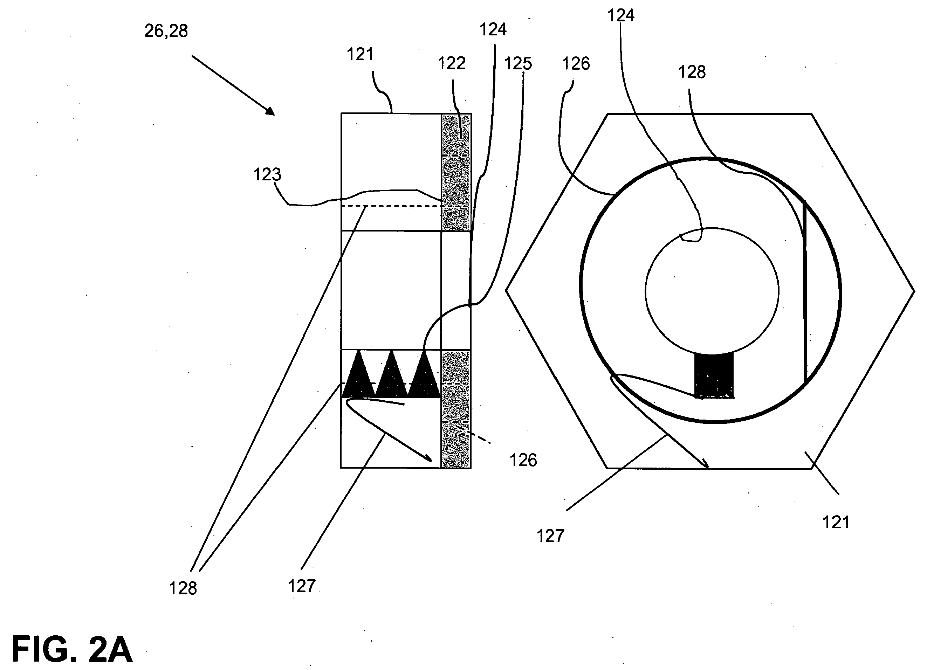

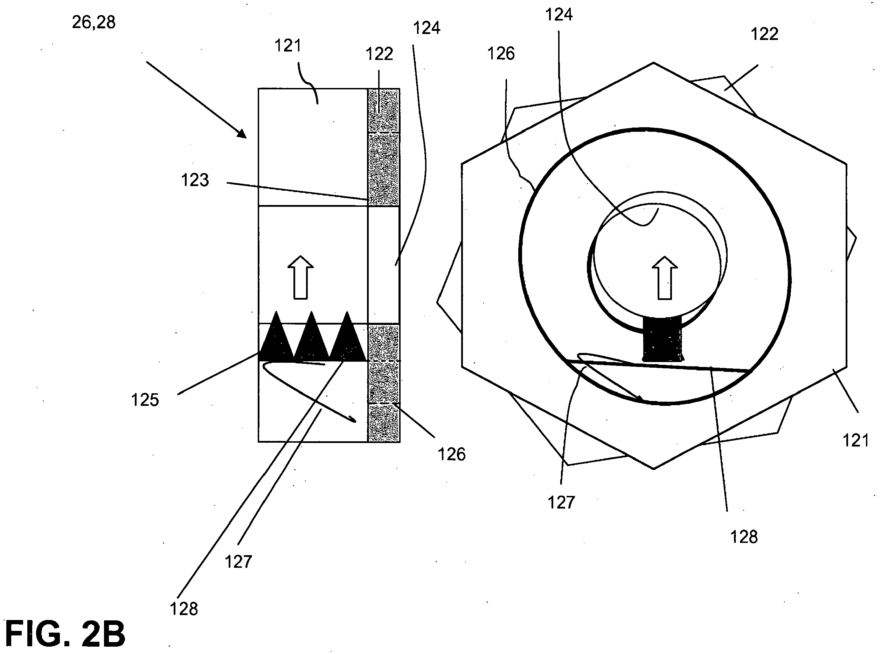

In Neutralstellung sitzt das Wirkelement 26 auf einem Sitz 20 und das Wirkelement 28 auf einem Sitz 22. Im Gehäusebereich 32 ragen die Wirkelemente 26, 28 aus dem Gehäuse 30 und sind von außen z.B. per Hand betätigbar. Deren Wirkprinzip ist in den Figuren 2a und 2b näher erläutert.

Da zwei Gewinde (Geschwindigkeitsbereiche 16, 18) und zwei Muttern (Wirkelemente 26, 28) vorhanden sind, kann ein Konflikt der Wirkelemente 26, 28 bei der Drehung vermieden werden, indem eines der Wirkelemente 26, 28 deaktiviert wird. Wenn z.B. das Wirkelement 26 gedreht und entlang des Geschwindigkeitsbereichs 16 bewegt wird, muss das Wirkelement 28 am anderen Geschwindigkeitsbereich 18 deaktiviert bzw. gelöst werden.

Hierfür sind die Wirkelemente 26, 28 speziell ausgebildet. Jedes Wirkelement 26, 28 besteht aus zwei axial miteinander verbundenen und radial mittels einer drehbaren Fixierung 123 gegeneinander verdrehbaren Teilen. Das erste Teil ist ein Körper 121 mit einem Bereich mit einem Innengewinde 125. Das Innengewinde 125 ist einer (zentralen) axialen Bohrung 124 im Körper 121 zugeordnet und kann aktiviert und gelöst (deaktiviert) werden. Die Bohrung 124 ist eine Durchgangsbohrung für die Spindel 10a (Figur 1). Das zweite Teil ist ringförmig ausgebildet und weist am inneren Umfang 126 eine Abflachung 128 auf. Das zweite Teil bildet eine Basis 122 ohne Innengewinde. Der Außenumfang der beiden Teile kann z.B. als Sechskant ausgebildet sein oder eine andere Form haben, z.B. ein Rändel oder dergleichen.

Der Durchmesser der Bohrung 124 ist so bemessen, dass die Spindel 10a mit seinem jeweiligen Geschwindigkeitsbereich 16, 18 mit dem Innengewinde 125 in aktivierter Position praktisch wie Mutter und Schraube zusammenwirken.

Das Innengewinde 125 ist auf die Steigung des Gewindes des jeweiligen Geschwindigkeitsbereichs 16 oder 18 abgestimmt und kann sich bereichsweise umlaufend um die Bohrung 124 erstrecken. Im dargestellten Ausführungsbeispiel ist das Innengewinde 125 nur über einen beschränkten Bereich des Innenumfangs der Bohrung 124 ausgebildet- Das Innengewinde 125 stellt praktisch nur ein Gewindestück dar. Eine Rückstellfeder 127 hält das Innengewinde 125 in einer Ruheposition außerhalb der Bohrung 124. In dieser Ruheposition ist das Wirkelement 16, 18 deaktiviert.

Die Basis 122 umfasst an seiner Innenseite eine Abflachung 128, welche sich durch die ganze axiale Länge des Körpers 121 erstreckt. Wird die Basis 122 gegenüber dem Körper 121 gedreht, gelangt die Abflachung 128 so unter das Innengewinde 125, dass dieses von der Abflachung 128 radial gegen die Federkraft der Rückstellfeder 127 in die Bohrung 124 gedrückt und durch die Abflachung 128 dort fixiert wird. Das Innengewinde 125 ist nunmehr in seinem aktivierten Zustand und kann jetzt in das Gewinde des zugehörigen Geschwindigkeitsbereichs 16, 18 eingreifen mit der Folge, dass sich beim Drehen des Körpers 121 gegenüber der Spindel 10a (Figur 1) die Einführelemente 52, 54 (Figur 1) axial gegeneinander bewegen. Die Abflachung 128 dient als Steuerfläche für die radiale Bewegung des Innengewindes 125. In dem dargestellten Ausführungsbeispiel (Figur 2b) wird die Basis 122 um 90° gedreht, um die Abflachung 128 vollständig unter das Innengewinde 125 zu drehen und das Innengewinde 125 in die lichte Bohrung 124 einzuführen.

Zur Deaktivierung des Wirkelements 26, 28 wird die Abflachung 128 zurückgedreht und das Innengewinde 125 somit radial freigegeben, so dass die Rückstellfeder 127 das Innengewinde 125 wieder radial nach außen bewegt und in die Ruhestellung gelangt. Bei einer Drehung des Wirkelements 26, 28 kann das Innengewinde 125 nunmehr nicht mehr in Eingriff mit dem entsprechenden Gewinde des Geschwindigkeitsbereichs 16, 18 gelangen. Das Wirkelement 26, 28 ist jetzt deaktiviert bzw. gelöst.

Im Falle eines Katheters als Einführvorrichtung mit einem Stent als Implantat kann in der Praxis der Stent anfangs mit langsamer Geschwindigkeit bis zu einer gewissen Länge freigesetzt und somit sehr präzise positioniert werden. Danach kann der Stent vollständig mit höherer Geschwindigkeit freigesetzt werden.

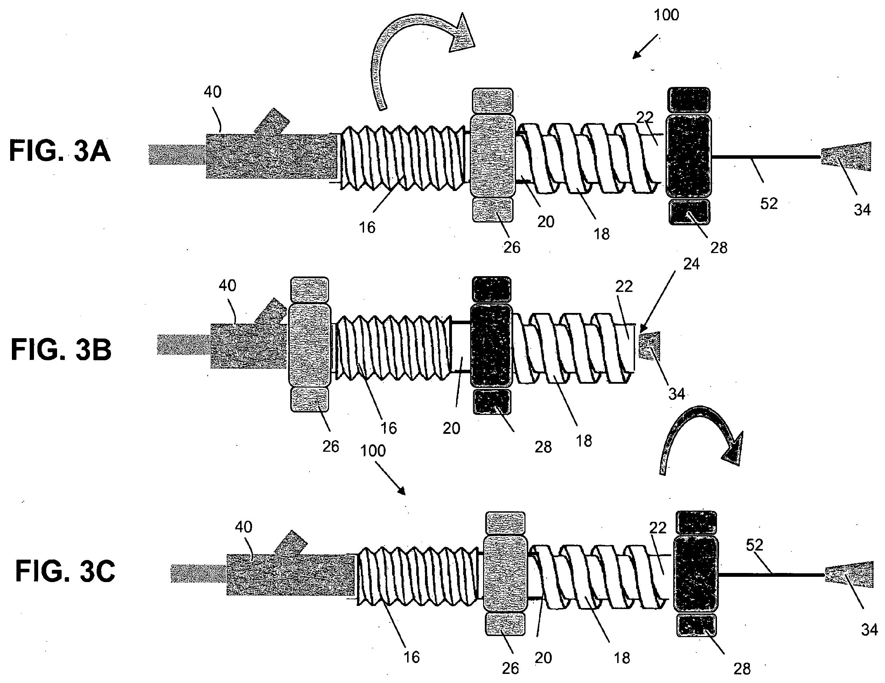

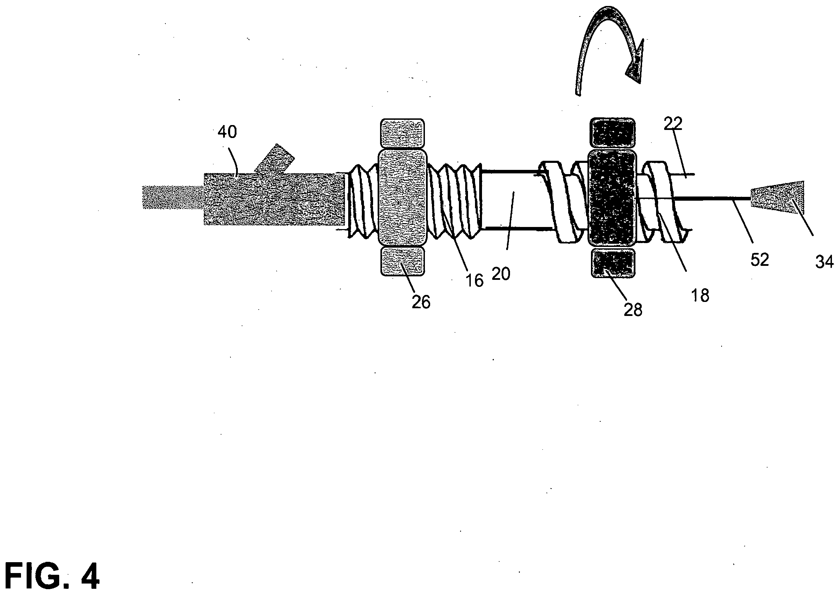

Die Figuren 3a bis 3c zeigen Zustände der Freigabevorrichtung 100 bzw. Positionen der Wirkelemente 26, 28 beim langsamen Freigeben des Implantats, und Figur 4 zeigt einen Zustand bzw. Positionen der Wirkelemente 26, 28 beim schnellen Freigeben des Implantats.

Zum langsamen Freigeben, d.h. um eine langsame Relativbewegung der beiden Einführelemente 52, 54 zu bewirken, ist zunächst das Wirkelement 26 zu aktivieren, d.h. das entsprechende Innengewinde radial nach innen zu bewegen, während das Wirkelement 28 deaktiviert sein muss, d.h. das entsprechende Innengewinde ist in seiner Neutralposition radial außerhalb der Durchgangsbohrung für die Spindel 10a positioniert Figur 3a). Wird das Wirkelement 26 um die Spindel 10a gedreht (Figur 3b), bewegt sich dieses wegen der geringen Gewindesteigung des Geschwindigkeitsbereichs 16 langsam entlang der Spindel 10a. bis das gewünschte Segment des Implantats freigegeben ist. Die Drehrichtung des Wirkelements 26 ist z.B. im Uhrzeigersinn, wie mit einem gekrümmten Pfeil über dem Wirkelement 26 angedeutet ist. Soll das ganze Implantat in voller Länge langsam freigegeben werden, wird das Wirkelement 26 solange weitergedreht, bis es den Spülkörper 40, der als Anschlag fungiert, erreicht. Das deaktivierte Wirkelement 28 gleitet derweilen ohne Gewindeeingriff auf der Spindel 10a.

Das langsame Freigeben eignet sich besonders für den Beginn der Implantatsfreigabe am Implantationsort.

Es ist auch möglich, vom langsamen zum schnellen Freigeben überzugehen. Dazu wird wie in Figur 3a das Wirkelement 26 für ein langsames Freigeben des Implantats aktiviert und das Wirkelement 28 deaktiviert. Das Wirkelement 26 wird um die Spindel 10a gedreht, bis ein gewünschter Teil der Gesamtlänge L16 des Geschwindigkeitsbereichs 16 zurückgelegt ist. Das Wirkelement 28 gleitet derweil funktionslos über die Spindel 10a. Dann wird das Wirkelement 28 für ein schnelles Freigeben des Implantats aktiviert und das Wirkelement 26 deaktiviert. Durch Drehen des Wirkelements 28 erfolgt nunmehr eine schnelle axiale Bewegung der Einführelemente 52, 54 (Figur 1).

Zum schnellen Freigeben wird das Wirkelement 28 aktiviert, d.h. das Innengewinde radial nach innen bewegt, während das Wirkelement 26 deaktiviert sein muss (Figur 4). Wird das Wirkelement 28 um die Spindel 10a gedreht, bewegen sich die Einführelement 52, 54 wegen der gro0en Gewindesteigung des Geschwindigkeitsbereichs 18 schneller relativ zueinander als bei der langsamen Freisetzung und werden schnell auseinander gezogen, was zur schnellen Freigabe des Implantats führt.

## Patentansprüche

1. Freigabevorrichtung (100) zum Lösen eines medizinisches Implantats von einer Einführvorrichtung (110), bei welcher das Implantat durch eine Relativbewegung zwischen einem ersten und einem zweiten Einführelement (52, 54) freisetzbar ist, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12, 14) eine Spindel (10a) mit wenigstens zwei Geschwindigkeitsbereichen (16, 18) vorgesehen ist, wobei die Spindel (10a) zur Erzeugung einer gezielten Relativbewegung zwischen dem ersten und dem zweiten Einführelement (52, 54) der Einführvorrichtung (110) vorgesehen ist,
**dadurch gekennzeichnet, dass**
dem jeweiligen Geschwindigkeitsbereich (16, 18) je ein Wirkelement (26, 28) zugeordnet ist, das mit dem jeweiligen Geschwindigkeitsbereich (16, 18) zusammenwirkt, um die Relativbewegung zwischen dem ersten und dem zweiten Einführelement (52, 54) der Einführvorrichtung (110) zu bewirken

2. Freigabevorrichtung nach Anspruch 1, wobei die Geschwindigkeitsbereiche (16, 18) jeweils entlang einer Längserstreckung (L) der Spindel (10), bevorzugt hintereinander, angeordnet sind.

3. Freigabevorrichtung nach einem der vorhergehenden Ansprüche, wobei die Geschwindigkeitsbereiche (16, 18) eine Gewindesteigung aufweisen, die für die verschiedenen Geschwindigkeitsbereiche (16, 18) unterschiedlich ist.

4. Freigabevorrichtung nach einem der vorhergehenden Ansprüche, wobei das Wirkelement (26, 28) gegenüber einem Gehäuse (30) fixiert ist, das wenigstens um die Spindel (10a) angeordnet ist.

5. Freigabevorrichtung nach Anspruch 4, wobei das jeweilige Wirkelement (26, 28) ein Betätigungselement (122) aufweist, das aus dem Gehäuse (30) ragt.

6. Freigabevorrichtung nach einem der vorhergehenden Ansprüche, wobei das Wirkelement (26, 28) wenigstens bereichsweise ein Gewinde (125) umfasst.

7. Freigabevorrichtung nach Anspruch 6, wobei das Gewinde (125) im Wirkelement (26, 28) radial verschiebbar ist.

8. Einführvorrichtung (110) zum Einführen eines medizinischen Implantats, welches durch eine Relativbewegung zwischen einem ersten und einem zweiten Einführelement (26, 28) freisetzbar ist, umfassend eine Freigabevorrichtung (100) zum Lösen des medizinisches Implantats nach einem der vorhergehenden Ansprüche, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (14) eine Spindel (10a) mit wenigstens zwei Geschwindigkeitsbereichen (16, 18) vorgesehen ist, wobei die Spindel (10a) zur Erzeugung einer gezielten Relativbewegung zwischen dem ersten und dem zweiten Einführelement (52, 54) der Einführvorrichtung (110) vorgesehen ist, und wobei dem jeweiligen Geschwindigkeitsbereich (16, 18) je ein Wirkelement (26, 28) zugeordnet ist, das mit dem jeweiligen Geschwindigkeitsbereich (16, 18) zusammenwirkt, um die Relativbewegung zwischen dem ersten und dem zweiten Einführelement (52, 54) der Einführvorrichtung (110) zu bewirken.

9. Einführvorrichtung nach Anspruch 8, wobei am distalen Ende (12) eine Schleusenvorrichtung (40) angeordnet ist, die mit einem äußeren der Einführelemente (54) verbunden ist.

10. Einführvorrichtung nach Anspruch 8 oder 9, wobei ein inneres (Einführelement (52) durch die Spindel (10a) geführt ist.

11. Einführvorrichtung nach Anspruch 9 oder 10, wobei die Spindel (10a) an der Schleusenvorrichtung (40) fixiert ist.

12. Einführvorrichtung nach einem der Ansprüche 8 bis 11, wobei das Wirkelement (26, 28) wenigstens ein Gewinde (125) aufweist, welches durch ein Betätigungselement (26a, 28a) radial im Wirkelement (26, 28) verschiebbar ist, und welches aus einem Gehäuse (30) ragt, das wenigstens um die Spindel (10a) angeordnet ist.

## Claims

1. A release device (100) for detaching a medical implant from an insertion device (110), in which the implant can be released by way of a relative movement between a first and a second insertion element (52, 54), comprising a body (10) having a proximal end (12), which faces a user in the usage state, and a distal end (14), which is remote from the user in the usage state, wherein a spindle (10a) comprising at least two speed regions (16, 18) is provided between the proximal and distal ends (12, 14), wherein the spindle (10a) is provided so as to generate a deliberate relative movement between the first and second insertion elements (52, 54) of the insertion device (110),
**characterised in that**
the respective speed region (16, 18) is associated with a respective operating element (26, 28) which cooperates with the respective speed region (16, 18) so as to effect the relative movement between the first and second insertion elements (52, 54) of the insertion device (110).

2. The release device according to Claim 1, wherein the respective speed regions (16, 18) are disposed along a longitudinal extension (L) of the spindle (10), preferably behind one another.

3. The release device according to any one of the preceding claims, wherein the speed regions (16, 18) have a thread pitch that is different for the different speed regions (16, 18).

4. The release device according to any one of the preceding claims, wherein the operating element (26, 28) is fixed with respect to a housing (30), which is disposed at least around the spindle (10a).

5. The release device according to Claim 4, wherein the respective operating element (26, 28) comprises an actuating element (122) protruding from the housing (30).

6. The release device according to any one of the preceding claims, wherein the operating element (26, 28) has a thread (125) at least in some regions.

7. The release device according to Claim 6, wherein the thread (125) can be radially displaced in the operating element (26, 28).

8. An insertion device (110) for inserting a medical implant, which can be released by way of a relative movement between a first and a second insertion element (26, 28), comprising a release device (100) for detaching the medical implant according to any one of the preceding claims, comprising a body (10) having a proximal end (12), which faces a user in the usage state, and a distal end (14), which is remote from the user in the usage state, wherein a spindle (10a) having at least two speed regions (16, 18) is provided between the proximal end and distal end (14), wherein the spindle (10a) is provided so as to generate a deliberate relative movement between the first and second insertion elements (52, 54) of the insertion device (110), and wherein the respective speed region (16, 18) is associated with a respective operating element (26, 28) which cooperates with the respective speed region (16, 18) so as to effect the relative movement between the first and second insertion elements (52, 54) of the insertion device (110).

9. The insertion device according to Claim 8, wherein a sheath device (40), which is connected to an outer one of the insertion elements (54), is disposed at the distal end (12).

10. The insertion device according to Claim 8 or 9, wherein an inner insertion element (52) is guided through the spindle (10a).

11. The insertion device according to Claim 9 or 10, wherein the spindle (10a) is fixed to the sheath device (40).

12. The insertion device according to one of Claims 8 to 11, wherein the operating element (26, 28) comprises at least one thread (125), which can be displaced radially in the operating element (26, 28) by means of an actuating element (26a, 28a) and which protrudes from a housing (30) disposed at least around the spindle (10a).

## Revendications

1. Dispositif de libération (100) pour le détachement d'un implant médical d'un dispositif d'implantation (110), dans lequel l'implant peut être libéré par un mouvement relatif entre un premier et un second éléments d'implantation (52, 54), comprenant un corps (10) avec une extrémité proximale (12), qui est orientée vers un utilisateur à l'état d'emploi, et une extrémité distale (14), qui est éloignée de l'utilisateur à l'état d'emploi, où une broche (10a) avec au moins deux domaines de vitesse (16, 18) est prévue entre les extrémités (12, 14) proximale et distale, où la broche (10a) est prévue pour la création d'un mouvement relatif ciblé entre les premier et second éléments d'implantation (52, 54) du dispositif d'implantation (110),
**caractérisé en ce**
**qu'**un élément actif (26,28) est associé à chaque domaine de vitesse (16, 18), élément qui agit conjointement avec le domaine de vitesse (16, 18) respectif afin de provoquer le mouvement relatif entre les premier et second éléments d'implantation (52, 54) du dispositif d'implantation (110).

2. Dispositif de libération selon la revendication 1, où les domaines de vitesse (16, 18) sont respectivement disposés le long d'une extension longitudinale (L) de la broche (10), de préférence l'un derrière l'autre.

3. Dispositif de libération selon l'une des revendications précédentes, où les domaines de vitesse (16, 18) présentent une augmentation de filetage qui est différente pour les divers domaines de vitesses (16, 18).

4. Dispositif de libération selon l'une des revendications précédentes, où l'élément actif (26, 28) est fixé en face d'un boitier (30) qui est disposé au moins autour de la broche (10a).

5. Dispositif de libération selon la revendication 4, où chaque élément actif (26, 28) présente un élément d'actionnement (122) qui dépasse du boitier (30).

6. Dispositif de libération selon l'une des revendications précédentes, où l'élément actif (26, 28) comprend au moins partiellement un filetage (125).

7. Dispositif de libération selon la revendication 6, où le filetage (125) peut être déplacé radialement dans l'élément actif (26, 28).

8. Dispositif d'implantation (110) pour l'insertion d'un implant médical, lequel peut être libéré par un mouvement relatif entre un premier et un second éléments d'implantation (26, 28), comprenant un dispositif de libération (100) pour le détachement de l'implant médical selon l'une des revendications précédentes, comprenant un corps (10) avec une extrémité proximale (12), qui est orientée vers un utilisateur à l'état d'emploi, et une extrémité distale (14), qui est éloignée de l'utilisateur à l'état d'emploi, où une broche (10a) avec au moins deux domaines de vitesse (16, 18) est prévue entre les extrémités (12, 14) proximale et distale, où la broche (10a) est prévue pour la création d'un mouvement relatif ciblé entre les premier et second éléments d'implantation (52, 54) du dispositif d'implantation (110), et où
un élément actif (26, 28) est associé à chaque domaine de vitesse (16, 18), élément qui agit conjointement avec le domaine de vitesse (16, 18) respectif afin de provoquer le mouvement relatif entre les premier et second éléments d'implantation (52, 54) du dispositif d'implantation (110).

9. Dispositif d'implantation selon la revendication 8, où un dispositif de verrouillage (40), qui est relié avec un élément extérieur parmi les éléments d'implantation (54), est disposé à l'extrémité distale (12).

10. Dispositif d'implantation selon les revendications 8 ou 9, où un élément d'implantation interne (52) est conduit par la broche (10a).

11. Dispositif d'implantation selon les revendications 9 ou 10, où la broche (10a) est fixée sur le dispositif de verrouillage (40).

12. Dispositif d'implantation selon l'une des revendications 8 à 11, où l'élément actif (26, 28) présente au moins un filetage (125), lequel peut être déplacé radialement dans l'élément actif (26, 28) par un élément d'actionnement (26a, 28a), et lequel dépasse d'un boitier (30) qui est disposé au moins autour de la broche (10a).
